# FASCICULE DE BREVET EUROPEEN

(11) **EP 1 637 186 B1**
(45) Date de publication et mention de la délivrance du brevet: **10.10.2007**
(21) Numéro de dépôt: 05291703.6
(22) Date de dépôt: 09.08.2005
(51) Int. Cl.: A61Q 1/02, A61Q 17/04, A61Q 19/00, A61K 8/06, A61K 8/25, A61K 8/81

(54) **Composition cosmétique matifiante sous forme d'émulsion huile-dans-eau contenant de la silice pyrogénée**
Mattierende kosmetische Zusammensetzung in Form einer Öl-in-Wasser Emulsion enthaltend pyrogene Kieselsäure
Matifying cosmetic composition in the form of an oil-in-water emulsion comprising pyrogenic silica

(30) Priorité: 23.08.2004 FR 0451884
(43) Date de publication de la demande: 22.03.2006
(73) Titulaire: L'ORÉAL, 75008 Paris (FR)
(72) Inventeur: Fonolla Moreno, Angeles, 75013 Paris (FR)
(74) Mandataire: Rasson, Catherine

(56) Documents cités:
- FR-A- 2 834 450
- FR-A- 2 834 461
- FR-A- 2 839 445

## Description

La présente demande concerne une composition cosmétique, sous forme d'émulsion huile-dans-eau contenant au moins un homopolymère d'AMPS et au moins une silice pyrogénée hydrophile. La demande se rapporte aussi à l'utilisation de ladite composition dans le domaine cosmétique, notamment pour l'hydratation de la peau et l'obtention d'un aspect mat de la peau.

On sait combien il est important pour la peau d'être bien hydratée car l'exposition de la peau à l'atmosphère entraîne une déshydratation qui doit être compensée par un apport d'eau ou d'actifs hydratants. Pour diverses raisons liées en particulier à un meilleur confort d'utilisation, les compositions cosmétiques se présentent le plus souvent sous la forme d'une émulsion du type huile-dans-eau (H/E) constituée d'une phase continue dispersante aqueuse et d'une phase discontinue dispersée huileuse, car ces émulsions, du fait qu'elles comportent comme phase externe, une phase aqueuse, présentent, lors de l'application sur la peau, un toucher plus frais, moins gras et plus léger que les émulsions inverses eau-dans-huile (E/H). Par ailleurs, pour avoir un effet hydratant et en même temps une sensation de fraîcheur, on cherche à préparer des émulsions H/E très légères dont la quantité de phase aqueuse soit importante et dont, par conséquent, la quantité de phase huileuse (ou phase grasse) soit limitée.

De manière préférée ces émulsions se présentent sous forme de crèmes, c'est-à-dire qu'elles sont suffisamment épaissies pour pouvoir être facilement prises au doigt sans couler, et aussi pour pouvoir bien s'étaler sur la peau. Le fait d'avoir la viscosité d'une crème donne aussi une sensation très agréable et un toucher très fondant lors de l'application du produit. La composition crémeuse s'applique de façon très homogène et la sensation de confort est bien ressentie. En outre, on cherche à ce que les crèmes de ce type aient des propriétés matifiantes. Par définition, un produit matifiant est un produit qui empêche la peau de briller et qui unifie le teint. Les compositions de soin de la peau ou de maquillage ayant des propriétés matifiantes sont généralement utilisées pour résoudre les problèmes de brillance occasionnés par un excès de sébum, et pour améliorer la tenue du maquillage à long terme, le maquillage ayant tendance à se dégrader visuellement au cours de la journée. Ces compositions donnent un aspect mat à la peau, résultant d'un pouvoir diffusant de la lumière à la surface de la peau. Elles peuvent aussi être utilisées pour estomper les défauts de la peau, tels que les microreliefs, les rides, les ridules, les pores ou les variations de couleur.

De façon générale, les compositions cosmétiques matifiantes sont formulées à l'aide de charges minérales (ZnO, SiO₂) enrobées ou non, et d'amidon. Toutefois, il est très difficile d'incorporer des charges matifiantes performantes dans des émulsions H/E comportant une quantité de phase huileuse limitée, car l'incorporation de ces charges entraîne alors une destabilisation de l'émulsion, et elle peut entraîner aussi des phénomènes de « peluchages » (boulochage des charges, qui se forme après l'application du produit).

L'objectif de l'invention est de pouvoir réaliser des émulsions huile-dans-eau stables, c'est-à-dire qui ne se déphasent ni ne présentent de relargage d'huile, ayant de bonnes propriétés hydratantes et matifiantes, et ce même en présence d'une quantité limitée de phase huileuse.

La demanderesse a découvert de façon inattendue que l'utilisation d'une association d'une silice pyrogénée hydrophile en une quantité minimum d'au moins 0,2 % en poids, et d'un homopolymère d'acide 2-acrylamido 2-méthylpropane sulfonique, permettait de réaliser des émulsions stables huile-dans-eau comportant un faible taux de phase huileuse et ayant à la fois de bonnes propriétés hydratantes et matifiantes.

Le document FR-A-2,834,461 décrit des compositions comprenant un copolymère siliconé bloc, une charge organique et une charge minérale qui peut être la silice, les compositions pouvant être des émulsions. Toutefois, ces compositions ne contiennent pas d'homopolymères d'acide 2-acrylamido 2-méthylpropane sulfonique.

La présente invention se rapporte à une composition pour application topique sous forme d'émulsion huile-dans-eau comprenant une phase huileuse dispersée dans une phase aqueuse, caractérisée en ce qu'elle contient (1) au moins 0,2 % en poids par rapport au poids total de la composition, d'une ou plusieurs silices pyrogénées hydrophiles, et (2) au moins un homopolymère d'acide 2-acrylamido 2-méthylpropane sulfonique, et en ce que la phase aqueuse représente au moins 70 % en poids par rapport au poids total de la composition.

La composition de l'invention présente l'avantage d'être stable et d'avoir de bonnes propriétés hydratantes et matifiantes. Elle peut constituer notamment une composition cosmétique. Du fait qu'elle est destinée à une application topique, la composition de l'invention contient un milieu physiologiquement acceptable. Par "milieu physiologiquement acceptable", on entend un milieu convenant à une application topique sur la peau ou les phanères, c'est-à-dire compatible avec la peau, les muqueuses, les lèvres, les cils, les yeux, les cheveux et les ongles. Cette composition peut constituer notamment une composition cosmétique ou dermatologique.

Les compositions selon l'invention se présentent sous forme de crèmes généralement assez épaisses, même pâteuses, ayant un aspect mat. La viscosité des compositions de l'invention est de préférence supérieure à 1 Pa.s, de préférence égale ou supérieure à 2 Pa.s, viscosité mesurée à 25°C avec l'appareil de mesure Rheomat 180 à 200 rpm (tours par minute). Le Rheomat 180 est équipé d'un mobile différent selon les viscosités, par exemple d'un mobile 3 pour la gamme des viscosités de 1 à 4 Pa.s, et d'un mobile 4 pour la gamme des viscosités supérieures à 2 Pa.s.. Cette viscosité peut aller par exemple de 1,5 à 30 Pa.s, de préférence de 2 à 20 Pa.s, mieux de 3 à 15 Pa.s et encore mieux de 5 à 10 Pa.s. Cette viscosité est mesurée généralement 10 minutes après la mise en rotation du mobile.

### Silice Pyrogénée hydrophile

Les silices pyrogénées utilisées dans la composition de l'invention sont hydrophiles.

Les silices hydrophiles pyrogénées sont obtenues par pyrolyse en flamme continue à 1000°C du tétrachlorure de silicium (SiCl₄) en présence d'hydrogène et d'oxygène. Parmi les silices pyrogénées à caractère hydrophile utilisables selon la présente invention, on peut citer notamment celles vendues par la société DEGUSSA HÜLS sous les dénominations commerciales AEROSIL® 90, 130, 150, 200, 300 et 380.

La quantité de silice(s) pyrogénée(s) doit être au moins de 0,2 % en poids par rapport au poids total de la composition. Cette quantité peut aller par exemple de 0,2 à 4 % en poids, de préférence de 0,3 à 3 % en poids, mieux de 0,4 à 2 % en poids et encore mieux de 0,5 à 1 % en poids par rapport au poids total de la composition.

### Homopolymère d'acide 2-acrylamido 2-méthylpropane sulfonique

On entend notamment dans la présente demande, par « homopolymère d'acide 2-acrylamido 2-méthylpropane sulfonique », les polymères comprenant comme monomères seulement de l'acide 2-acrylamido 2-méthylpropane sulfonique, et éventuellement des agents de réticulation. Ce sont des polymères hydrosolubles, et ils sont de préférence réticulés.

De façon préférentielle, ces polymères utilisés conformément à l'invention peuvent être neutralisés partiellement ou totalement par une base minérale (soude, potasse, ammoniaque) ou une base organique telle que la mono-, di- ou tri-éthanolamine, un aminométhylpropanediol, la N-méthyl-glucamine, les acides aminés basiques comme l'arginine et la lysine, et les mélanges de ces composés. Ils sont généralement neutralisés. On entend dans la présente invention par « neutralisés » des polymères totalement ou pratiquement totalement neutralisés, c'est-à-dire neutralisés à au moins 90 %.

Les agents de réticulation de ces polymères peuvent être choisis parmi les composés à polyinsaturation oléfinique couramment utilisés pour la réticulation des polymères obtenus par polymérisation radicalaire. On peut citer par exemple comme agents de réticulation, le divinylbenzène, l'éther diallylique, le dipropylèneglycol-diallyléther, les polyglycol-diallyléthers, le triéthylèneglycol-divinyléther, l'hydroquinone-diallyl-éther, le di(méth)acrylate d'éthylèneglycol ou de tétraéthylèneglycol, le triméthylol propane triacrylate, le méthylène-bis-acrylamide, le méthylène-bis-méthacrylamide, la triallylamine, le triallylcyanurate, le diallylmaléate, la tétraallyléthylènediamine, le tétra-allyloxy-éthane, le triméthylolpropane-diallyléther, le (méth)acrylate d'allyle, les éthers allyliques d'alcools de la série des sucres, ou d'autres allyl- ou vinyl- éthers d'alcools polyfonctionnels, ainsi que les esters allyliques des dérivés de l'acide phosphorique et/ou vinylphosphonique, ou les mélanges de ces composés.

Selon un mode préféré de réalisation de l'invention, l'agent de réticulation est choisi parmi le méthylène-bis-acrylamide, le méthacrylate d'allyle ou le triméthylol propane triacrylate (TMPTA). Le taux de réticulation va en général de 0,01 à 10 % en mole et plus particulièrement de 0,2 à 2 % en mole par rapport au polymère.

Ces homopolymères peuvent être obtenus selon le procédé de préparation comprenant les étapes suivantes :
(a) on disperse ou on dissout le monomère (acide 2-acrylamido 2-méthylpropane sulfonique) sous forme libre dans une solution de tertio-butanol ou d'eau et de tertio-butanol ;
(b) on neutralise la solution ou la dispersion de monomère obtenue en (a) par une ou plusieurs bases minérales ou organiques, de préférence l'ammoniaque NH₃, dans une quantité permettant d'obtenir un taux de neutralisation des fonctions acides sulfoniques du polymère allant de 90 à 100% ;
(c) on ajoute à la solution ou dispersion obtenue en (b), le ou les monomères réticulants;
(d) on effectue une polymérisation radicalaire classique en la présence d'amorceurs de radicaux libres à une température allant de 10 à 150°C ; le polymère précipitant dans la solution ou la dispersion à base de tertio-butanol.

Comme homopolymères de ce type, on peut citer notamment l'homopolymère réticulé et neutralisé d'acide 2-acrylamido 2-méthylpropane sulfonique, commercialisé par la société Clariant sous la dénomination commerciale « Hostacerin AMPS » (nom CTFA : ammonium polyacryldimethyltauramide).

De manière préférée, ces homopolymères qui sont hydrosolubles,sont introduits dans la phase aqueuse de l'émulsion selon l'invention.

L'homopolymère utilisé dans la composition de l'invention est de préférence présent en une quantité (en matière active) allant de 0,5 à 3 % en poids, mieux de 1 à 2 % en poids et encore mieux de 1 à 1,5 % en poids par rapport au poids total de la composition.

### Phase huileuse

La phase huileuse appelée aussi phase grasse, est constituée des constituants lipophiles, c'est-à-dire huiles et autres corps lipophiles présents dans la composition, ainsi que de tous additifs lipophiles éventuellement présents.

La quantité de phase huileuse dans la composition de l'invention est inférieure ou égale à 30 % en poids. Elle peut aller par exemple de 1 à 30 % en poids, de préférence de 2 à 25 % et mieux de 2 à 15 % en poids par rapport au poids total de la composition.

La phase huileuse contient au moins une huile, notamment une huile cosmétique. On entend par "huile" un corps gras liquide à la température ambiante (25°C).

Comme huiles plus particulièrement utilisables dans la composition de l'invention, on peut citer par exemple :
- les huiles hydrocarbonées d'origine animale, telles que le perhydrosqualène (ou squalane) ;
- les huiles hydrocarbonées d'origine végétale, telles que les triglycérides liquides d'acides gras, par exemple les huiles de tournesol, de maïs, de soja, de courge, de pépins de raisin, de sésame, de noisette, d'abricot, de macadamia, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel ;
- les esters et les éthers de synthèse, notamment d'acides gras, comme les huiles de formules R¹COOR² et R¹OR² dans laquelle R¹ représente le reste d'un acide gras comportant de 8 à 29 atomes de carbone, et R² représente une chaîne hydrocarbonée, ramifiée ou non, contenant de 3 à 30 atomes de carbone, comme par exemple l'huile de Purcellin, le laurate d'hexyle, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; les esters du pentaérythritol comme le tétraisostéarate de pentaérythrytyle ; les dérivés lipophiles d'acides aminés, tels que le lauroyl sarcosinate d'isopropyle (nom INCI : Isopropyl Lauroyl sarcosinate) commercialisé sous la dénomination Eldew SL 205 par la société Ajinomoto ;
- les hydrocarbures linéaires ou ramifiés, d'origine minérale ou synthétique, tels que les huiles minérales (mélange d'huiles hydrocarbonées dérivées du pétrole ; nom lNCl : Mineral oil), les huiles de paraffine, volatiles ou non, et leurs dérivés, la vaseline, les polydécènes, l'isohexadecane, l'isododecane, l'isoparaffine hydrogéné tel que l'huile de Parléam® commercialisée par la société NOF Corporation (nom lNCl ; Hydrogenated Polyisobutene) ;
- les huiles de silicone comme les polyméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que la cyclopentasiloxane et la cyclohexadiméthylsiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, et les polyméthylphénylsiloxanes ;
- les huiles fluorées telles que celles partiellement hydrocarbonées et/ou siliconées comme celles décrites dans le document JP-A-2-295912 ;
- les éthers tels que l'éther dicaprylique (nom CTFA : Dicaprylyl ether) ; et les benzoates d'alcools gras en C₁₂-C₁₅ (Finsolv TN de FINETEX) ;
- leurs mélanges.

Selon un mode préféré de réalisation de l'invention, la phase huileuse contient au moins une huile de silicone volatile, et notamment une cyclométhicone choisie par exemple parmi la cyclopentasiloxane, la cyclohexadiméthylsiloxane et leurs mélanges.

### Phase aqueuse

La composition selon l'invention comprend une quantité de phase aqueuse égale ou supérieure à 70 % du poids total de la composition, cette quantité pouvant aller par exemple de 70 à 99 % en poids et de préférence de 70 à 95 % en poids par rapport au poids total de la composition. La quantité d'eau dans la phase aqueuse peut aller par exemple de 50 à 80 % en poids et de préférence de 60 à 75 % en poids par rapport au poids total de la composition.

De manière classique, la phase aqueuse peut contenir, outre l'eau, un ou plusieurs solvants hydrosolubles choisis parmi les polyols (ou alcools polyhydriques), les alcool(s) inférieur(s) hydrosoluble(s) et leurs mélanges. On entend par alcool inférieur, un alcool comportant de 1 à 8 atomes de carbone.

Comme polyols, on peut citer par exemple la glycérine ; les glycols comme le pentylène glycol, le propylène glycol, le butylène glycol, l'isoprène glycol et les polyéthylène glycols tels que le PEG-8 ; le sorbitol ; les sucres tels que le glucose, le fructose, le maltose, le lactose, le sucrose ; et leurs mélanges. Comme alcools inférieurs, on peut citer par exemple l'éthanol, l'isopropanol, le butanol et leurs mélanges. Lorsqu'ils sont présents dans la composition de l'invention, le ou les solvant(s) peuvent être en une quantité allant de 0,01 à 60 % en poids, de préférence de 0,5 à 50 % en poids et mieux de 5 à 20 % en poids par rapport au poids total de la phase aqueuse. Ces adjuvants ainsi que leurs concentrations doivent être tels qu'ils ne modifient pas la propriété recherchée pour la composition de l'invention.

Selon un mode préféré de réalisation de l'invention, la phase aqueuse contient au moins un alcool inférieur, qui peut être notamment l'éthanol. La quantité d'alcool inférieur tel que l'éthanol peut aller par exemple de 2 à 30 % en poids et de préférence de 5 à 10 % en poids par rapport au poids total de la composition.

Le pH de la composition est compatible avec la peau et va de préférence de 5 à 7,5 et mieux de 5,5 à 7.

### Emulsionnants

L'émulsion selon l'invention contient de préférence un ou plusieurs émulsionnants choisis parmi ceux habituellement utilisés pour la préparation des émulsions H/E.

Comme émulsionnants des émulsions H/E, on peut citer par exemple les émulsionnants non ioniques, et en particulier :
- les esters d'acides gras et de polyols oxyalkylénés (plus particulièrement polyoxyéthylénés), et par exemple les stéarates de polyéthylène glycol comme le stéarate de PEG-100, le stéarate de PEG-50 et le stéarate de PEG-40 ; et leurs mélanges tels que le mélange de mono-stéarate de glycéryle et de stéarate de polyéthylène glycol (100 OE) commercialisé sous la dénomination SIMULSOL 165 par la société SEPPIC ;
- les esters d'acides gras et de sorbitan oxyalkylénés comprenant par exemple de 20 à 100 OE, et par exemple ceux commercialisés sous les dénominations commerciales Tween 20 ou Tween 60 par la société Ubiqema ;
- les éthers d'alcools gras oxyalkylénés (oxyéthylénés et/ou oxypropylénés) ;
- les esters de sucres, alcoxylé ou non, comme le stéarate de sucrose et comme le PEG-20 méthylglucose sesquistéarate ;
- les esters de sorbitan tels que le palmitate de sorbitan commercialisé sous la dénomination Span 40 par la société Uniqema ;
- les esters de diacide et d'alcool gras, tels que le tartrate de dimyristyle ; les mélanges de ces émulsionnants comme par exemple le mélange de stéarate de glycéryle et de stéarate de PEG-100, commercialisé sous la dénomination Arlacel 165 par la société Uniqema ; et les mélanges contenant ces émulsionnants, tels que le mélange de tartrate de dimyristyle, d'alcool cétéarylique, de Pareth-7 et de PEG-25 laureth-25, commercialisé sous la dénomination Cosmacol PSE par la société Sasol (nom CTFA : Dimyristyl tartrate / cetearyl alcool /12-15 Pareth 7 / PPG 25 laureth 25) ;
- les alkylpolyglucosides (APG) dont la chaîne alkyle est linéaire ou ramifiée et comprend de 12 à 22 atomes de carbone, seuls ou en mélange avec un alcool gras linéaire ou ramifié, ayant de 12 à 22 atomes de carbone. Les mélanges émulsionnants alcool gras/alkylpolyglycoside sont décrits dans les demandes WO92/06778, WO95/13863 et WO98/47610 et ils peuvent être préparés selon les procédés de préparation indiqués dans ces documents. Comme mélanges alcool gras/alkylpolyglycoside particulièrement préférés, on peut citer les produits vendus par la société SEPPIC sous les appellations MONTANOV ® tels que les mélanges Alcool cétylstéarylique/Cocoglucoside (MONTANOV 82®), Alcool arachidylique et alcool béhénylique/arachidylglucoside (MONTANOV 202®), Alcool myristylique/Myristylglucoside (MONTANOV 14 ®), Alcool cétylstéarylique/Cétylstéarylglucoside (MONTANOV 68 ®), Alcool en C₁₄-C₂₂/C₁₂₋ C₂₀ alkylglucoside (MONTANOV L ®), Cocoalcool /Coco-glucoside (MONTANOV S ®), et Alcool isostéarylique/Isostéarylglucoside (MONTANOV WO 18®).

Selon un mode préféré de réalisation de l'invention, l'émulsionnant est choisi parmi les alkylpolyglucosides, et notamment parmi ceux en mélange avec un alcool gras linéaire ou ramifié, en C12 à C22 tels que les Montanov.

De manière générale, on peut ajouter à ces émulsionnants, des co-émulsionnants tels que par exemple les alcools gras ayant de 8 à 26 atomes de carbone, comme l'alcool cétylique, l'alcool stéarylique et leur mélange (alcool cétéarylique), l'octyldodécanol, le 2-butyloctanol, le 2-hexyldécanol, le 2-undécylpentadécanol ou l'alcool oléique.

Le ou les émulsionnants sont présents en une quantité pouvant aller par exemple de 0,5 à 10 % en poids, mieux de 1 à 10 % en poids et encore mieux de 2 à 5 % en poids par rapport au poids total de la composition. La quantité de co-émulsionnants peut aller par exemple de 0,1 à 2 % en poids et mieux de 0,3 à 1 % en poids par rapport au poids total de la composition.

### Additifs

La composition selon l'invention peut aussi contenir tout adjuvant ou additif habituellement utilisé dans les domaines considérés et notamment dans les domaines cosmétique ou dermatologique.

Parmi les adjuvants classiques susceptibles d'être contenus dans la phase aqueuse et/ou dans la phase lipophile des émulsions conformes à l'invention (selon le caractère hydrosoluble ou liposoluble de ces adjuvants), on peut citer notamment les conservateurs ; les séquestrants (EDTA) ; les antioxydants ; les parfums ; les matières colorantes telles que les colorants solubles, les pigments et les nacres ; les charges matifiantes, tenseurs, blanchissantes ou exfoliantes ; les filtres solaires ; les actifs cosmétiques ou dermatologiques hydrophiles ou lipophiles ; les polymères ; les électrolytes. Les quantités de ces différents adjuvants sont celles classiquement utilisées dans le domaine considéré, et par exemple de 0,01 à 20 % du poids total de la composition.

Comme actifs utilisables dans la composition de l'invention, on peut citer par exemple, les vitamines hydrosolubles ou liposolubles comme la vitamine A (rétinol), la vitamine E (tocophérol), la vitamine C (acide ascorbique), la vitamine B5 (panthénol), la vitamine B3 (niacinamide), les dérivés de ces vitamines (notamment esters) et leurs mélanges ; les antiseptiques ; les actifs anti-bactériens comme le 2,4,4'-trichloro-2'-hydroxy diphényl éther (ou triclosan), le 3,4,4'-trichlorocarbanilide (ou triclocarban) ; les antisébohrréïques ; les antimicrobiens tels que le peroxyde de benzoyle, l'acide salicylique, le triclosan, l'acide azélaïque, la niacine (vit. PP) ; les amincissants tels que la caféine ; les azurants optiques, et tout actif approprié pour le but final de la composition, et leurs mélanges.

La quantité d'actifs dépend du but recherché. Le ou les actifs peuvent être par exemple présents en une concentration allant de 0,001 à 20 %, de préférence de 0,01 à 10 % en poids et mieux de 0,05 à 5 % du poids total de la composition.

Comme charges autres que la silice pyrogénée, on peut citer les poudres (organiques ou minérales) et les fibres.

Comme poudres qui peuvent être utilisées dans la composition de l'invention, on peut citer par exemple, les oxydes de zinc ; les oxydes de titane tels que le dioxyde de titane ; les micas d'origine naturelle ou synthétique ; le carbonate de calcium ; le carbonate et l'hydrocarbonate de magnésium ; les microbilles de silice telles que celles commercialisées sous la dénomination SB150 par la société Myoshi ; le talc ; le kaolin ; les billes de verre et de céramique commercialisées par la société 3M sous la dénomination commerciale MACROLITE ; les savons métalliques dérivés d'acide organique carboxylique ayant de 8 à 22 atomes de carbone, de préférence de 12 à 18 atomes de carbone, par exemple le stéarate de zinc, de magnésium ou de lithium, le laurate de zinc, le myristate de magnésium ; les particules de polyamide et notamment celles vendues sous les dénominations ORGASOL par la société Atochem ; les poudres de polymères synthétiques non expansées, telles que les poudres de polyéthylène, de polystyrène, de polyesters, de polyamides (par exemple le nylon ou la poly-β-alanine), de copolymères d'acrylates (par exemple les microsphères microporeuses vendues par la société Dow Corning sous la dénomination commerciale POLYTRAP), d'acides polyméthacryliques, de Téflon^{®} (polytétrafluuoroéthylène) comme les produits commercialisés sous les dénominations FLUON par la société Uniqema ; les poudres expansées telles que les microsphères creuses en matériau thermoplastique préparées par des procédés connus, comme ceux décrits dans les documents US-A-3 615 972 et

EP-A-0 56219, et notamment, les microsphères commercialisées sous les dénominations EXPANCEL par la société Kemanord Plast ou sous la dénomination MICROPEARL F 80 ED par la société Matsumoto ; les poudres de matériaux organiques naturels tels que les amidons de maïs, de blé ou de riz, réticulés ou non, telles que les poudres d'amidon réticulé par l'anhydride octénylsuccinate, commercialisées sous la dénomination DRY-FLO par la société National Starch ; les microbilles de résine de silicone telles que celles commercialisées sous la dénomination TOSPEARL par la société Toshiba Silicone ; et leurs mélanges.

Les filtres solaires pouvant être utilisés dans la composition de l'invention peuvent être des filtres organiques ou physiques. Comme exemples de filtres organiques, actifs dans l'UV-A et/ou l'UV-B, pouvant être ajoutés dans la composition de l'invention, on peut citer par exemple, les dérivés à fonction sulfonique tels que les dérivés sulfonés ou sulfonatés du benzylidène camphre, de la benzophénone ou du phénylbenzimidazole, plus particulièrement les dérivés du benzylidène camphre, comme l'acide benzène 1,4 [di(3-méthylidène-campho 10-sulfonique)], (nom INCI : Terephthalylidene Dicamphor Sulfonic Acid) fabriqué sous le nom MEXORYL SX par la société CHIMEX. l'acide 4'-sulfo 3-benzylidènecamphre (nom INCI : Benzylidene Camphor Sulfonic Acid), fabriqué sous le nom MEXORYL SL par la société CHIMEX, l'acide 2-[4-(camphométhylidène) phényl] benzimidazole-5-sulfonique, l'acide phénylbenzimidazole sulfonique (nom INCI : Phenylbenzimidazole Sulfonic Acid), commercialisé sous le nom EUSOLEX 232 par la société MERCK ; les dérivés de l'acide para-aminobenzoique ; les dérivés salicyliques tels que le salicylate d'éthyl hexyle vendu sous le nom commercial NEO HELIOPAN OS par Haarmann et Reimer ; les dérivés du dibenzoylméthane tels que le Butyl Methoxydibenzoylmethane vendu notamment sous le nom commercial PARSOL 1789 par Hoffmann La Roche ; les dérivés cinnamiques tels que l'éthylhexyl Methoxycinnamate vendu notamment sous le nom commercial PARSOL MCX par Hoffmann La Roche ; les dérivés de β,β'-diphénylacrylate tels que l'octocrylene (α-cyano-β,β-diphénylacrylate de 2-éthylhexyle) vendu sous le nom commercial UVINUL N539 par la société BASF ; les dérivés de la benzophénone tels que la Benzophenone-1 vendu sous le nom commercial UVINUL 400 par BASF, la Benzophenone-2 vendu sous le nom commercial UVINUL D50 par BASF, la Benzophenone-3 ou Oxybenzone, vendu sous le nom commercial UVINUL M40 par BASF, la Benzophenone-4 vendu sous le nom commercial UVINUL MS40 par BASF ; les Dérivés du benzylidène camphre tels que le 4-Methylbenzylidene camphre vendu sous le nom commercial EUSOLEX 6300 par MERCK ; les dérivés du phenyl benzimidazole tels que le Benzimidazilate vendu sous le nom commercial NEO HELIOPAN AP par Haarmann et Reimer ; les dérivés de la triazine tels que l'Anisotriazine vendu sous le nom commercial TINOSORB S par CIBA GEIGY et l'éthylhexyl triazone vendu notamment sous le nom commercial UVINUL T150 par BASF ; les dérivés du phenyl benzotriazole tels que Drometrizole Trisiloxane vendu sous le nom commercial SILATRIZOLE par Rhodia Chimie et le Méthylène bis-Benzotriazolyl Tetramethylbutylphénol, vendu sous forme solide sous le nom commercial MIXXIM BB/100 par FAIRMOUNT CHEMICAL ou sous forme micronisé en dispersion aqueuse sous le nom commercial TINOSORB M par CIBA SPECIALTY CHEMICALS ; les dérivés anthraniliques tels que le Menthyl anthranilate vendu sous le nom commercial NEO HELIOPAN MA par Haarmann et Reimer ; les dérivés d'imidazolines ; les dérivés du benzalmalonate ; et leurs mélanges.

Comme filtres physiques pouvant être ajoutés dans la composition de l'invention, on peut citer par exemple les pigments et nanopigments d'oxydes métalliques, enrobés ou non enrobés, notamment les oxydes de titane, de fer, de zirconium, de zinc ou de cérium, et leurs mélanges, ces oxydes pouvant être sous forme de micro- ou nanoparticules (nanopigments), éventuellement enrobées.

Bien entendu, l'homme de l'art veillera à choisir le ou les éventuels additifs de la composition selon l'invention de manière telle que les propriétés avantageuses attachées intrinsèquement à la composition conforme à l'invention ne soient pas ou substantiellement pas, altérées par l'addition envisagée.

La composition de l'invention peut être notamment utilisée en application topique, en particulier dans les domaines cosmétique et dermatologique.

La composition de l'invention trouve son application notamment dans un grand nombre de traitements cosmétiques de la peau, y compris le cuir chevelu, notamment pour le traitement, la protection, le soin de la peau, et/ou pour le maquillage de la peau. Elle donne notamment une bonne hydratation à la peau et a des propriétés matifiantes, donnant un effet mat à la peau.

La composition selon l'invention peut en particulier être utilisée comme produit de soin de la peau (y compris le cuir chevelu), notamment pour l'hydratation de la peau. Elle peut être aussi utilisée comme produit de maquillage de la peau après incorporation de charges ou de colorants ou pigments, ou comme produits solaires après incorporation de filtres.

Aussi, l'invention a encore pour objet l'utilisation cosmétique de la composition telle que définie ci-dessus pour le soin de la peau, et/ou pour le maquillage de la peau et/ou pour la protection solaire de la peau.

L'invention a aussi pour objet un procédé de traitement cosmétique de la peau, y compris du cuir chevelu, caractérisé par le fait qu'on applique sur la peau, une composition telle que définie ci-dessus.

L'invention a encore pour objet l'utilisation cosmétique de la composition telle que définie ci-dessus pour l'hydratation de la peau.

L'invention a encore pour objet l'utilisation cosmétique de la composition telle que définie ci-dessus, comme agent matifiant de la peau, c'est-à-dire pour donner un aspect mat à la peau.

Un tel effet matifiant convient à toutes les peaux, en particulier normales, mixtes ou grasses. Elle est particulièrement approprié pour les peaux grasses pour lesquelles un effet mat est souvent recherché. Aussi, l'invention a aussi pour objet l'utilisation cosmétique de la composition telle que définie ci-dessus pour le traitement des peaux grasses.

L'invention se rapporte à un procédé de traitement cosmétique de la peau, destiné à lui apporter un aspect mat, à estomper les imperfections du relief de la peau, en particulier à camoufler les microreliefs, les rides et les ridules, les pores, caractérisée en ce qu'on applique sur la peau, une composition telle que définie ci-dessus.

Les exemples qui suivent permettront de mieux comprendre l'invention, sans toutefois présenter un caractère limitatif. Les quantités indiquées sont en % en poids, sauf mention contraire.

| Composition | Exemple 1 selon l'invention | Exemple 2 selon l'invention | Exemple comparatif 1 | Exemple comparatif 2 | Exemple comparatif 3 |
|---|---|---|---|---|---|
| **PHASE A** | | | | | |
| Montanov 202 | 2 | 1.8 | 2 | 2 | 2 |
| Glyceryl stearate | 2 | 2 | 2 | 2 | 2 |
| Cyclohexasiloxane | 7 | 5 | 7 | 7 | 7 |
| Hexyl laureate | | 1 | | | |
| Vitamine E | 0,5 | 0,5 | 0,5 | 0,5 | 0,5 |
| Parfum | 0,2 | 0,2 | 0,2 | 0,2 | 0,2 |
| conservateur | 0,25 | 0,25 | 0,25 | 0,25 | 0,25 |
| | | | | | |

| **Phase B** | | | | | |
|---|---|---|---|---|---|
| Glycérine | 5 | 6 | 5 | 5 | 5 |
| conservateur | 0,2 | 0,2 | 0,2 | 0,2 | 0,2 |
| Pentylene glycol | 1 | 1 | 1 | 1 | 1 |
| Acide salicylique | 0,5 | 0,5 | 0,5 | 0,5 | 0,5 |
| Eau d'hammamelis | 0,8 | 0,8 | 0,8 | 0,8 | 0,8 |
| Hostacerin AMPS | 1 | 1,2 | | | |
| Carbomer | | | 0,15 | 0,15 | 0,05 |
| Pemulen TR-1 | | | | 0,4 | 0,3 |
| Pemulen TR-2 | | | | | 0,4 |
| EAU | Qs 100 | Qs 100 | Qs 100 | Qs 100 | Qs 100 |
| | | | | | |

| **Phase C** | | | | | |
|---|---|---|---|---|---|
| Triethanolamine | | | 0.7 | 0.84 | |
| | | | | | |

| **Phase D** | | | | | |
|---|---|---|---|---|---|
| Silice pyrogénée (Aerosil 200) | 0.6 | 0.4 | 0.6 | 0.6 | 0.6 |
| Talc | 1% | 3% | 1% | 1% | 1% |
| | | | | | |

| **Phase E** | | | | | |
|---|---|---|---|---|---|
| Ethanoll | 6 | 5 | 6 | 6 | 6 |
| Test de stabilité | Emulsion fine et serrée | Emulsion fine et serrée | Relargage à T0 | Relargage à T2 jours | Lâchage sur les bords à T0 et relargage après 15 jours |
| CONCLUSION | Stable | Stable | Pas stable | Pas stable | Pas stable |

Les exemples comparatifs montrent que seuls l'association de silice pyrogénée et de polymère d'AMPS permet d'obtenir une émulsion stable.

Les exemples 1 et 2 selon l'invention sont des crèmes blanches pâteuses à l'aspect mat, qui s'étalent bien. Ces compositions ont fait l'objet de différents tests qui ont confirmé de très bonnes performances cosmétiques, c'est-à-dire :
**⇒** Un effet matifiant immédiat a été constaté. Cet effet est significatif jusqu'à 8 heures après application.
⇒ Les pores sont visiblement resserrés à T0 (temps zéro) et après 4 semaines de traitement.
**⇒** Les compositions sont hydratantes avec un effet significatif jusqu'à 24 heures.

### Par ailleurs, les exemples comparatifs suivants ont été faits :

Exemple comparatif 4 : dans l'exemple 1, on a remplacé l'Hostacerin AMPS par une quantité équivalente de Sepigel 305 (émulsion E/H de copolymère anionique réticulé d'acrylamide et d'acide 2-acrylamido 2-méthylpropane sulfonique (nom C.T.F.A. : Polyacrylamide / C13-14 Isoparaffin / Laureth-7), on a observé une chute de viscosité et on obtient une composition très fluide qui a de plus un toucher assez collant. La viscosité de l'exemple 1 est de 78 poises (7,8 Pa.s) alors que celle de l'exemple comparatif 4 est de 24 poises (2,4 Pa.s).

Exemple comparatif 5 : dans l'exemple 1, on a remplacé l'Aerosil 200 par des microsphères de silice amorphe, non pyrogénée (Sunsphere H51). La crème obtenue est plus fluide que celle de l'exemple 1 et n'a pas l'aspect mat de la crème pâteuse de l'exemple 1. En outre elle ne donne pas d'effet matifiant.

## Revendications

1. Composition pour application topique sous forme d'émulsion huile-dans-eau comprenant une phase huileuse dispersée dans une phase aqueuse, **caractérisée en ce qu'**elle contient (1) au moins 0,2 % en poids par rapport au poids total de la composition, d'une ou plusieurs silices pyrogénées hydrophiles, et (2) au moins un homopolymère d'acide 2-acrylamido 2-méthylpropane sulfonique, et **en ce que** la phase aqueuse représente au moins 70 % en poids par rapport au poids total de la composition.

2. Composition selon la revendication 1, **caractérisée en ce qu'**elle a une viscosité mesurée à 25°C supérieure à 1 Pa.s, viscosité mesurée à 25°C avec l'appareil de mesure Rheomat 180 à 200 tours par minute..

3. Composition selon l'une quelconque des revendications précédentes, **caractérisée en ce que** la quantité de silice(s) pyrogénée(s) va de 0,2 à 4 % en poids par rapport au poids total de la composition.

4. Composition selon l'une quelconque des revendications précédentes, **caractérisée en ce que** l'homopolymère d'acide 2-acrylamido 2-méthylpropane sulfonique est un homopolymère réticulé et neutralisé d'acide 2-acrylamido 2-méthylpropane sulfonique.

5. Composition selon l'une quelconque des revendications précédentes, **caractérisée en ce que** l'homopolymère d'acide 2-acrylamido 2-méthylpropane sulfonique est présent en une quantité allant de 0,5 à 3 % en poids par rapport au poids total de la composition.

6. Composition selon l'une quelconque des revendications précédentes, **caractérisée en ce que** la quantité de phase huileuse va de 1 à 30 % en poids par rapport au poids total de la composition.

7. Composition selon l'une quelconque des revendications précédentes, **caractérisée en ce que** la phase huileuse contient au moins une huile de silicone volatile.

8. Composition selon l'une quelconque des revendications précédentes, **caractérisée en ce que** la phase aqueuse contient au moins un alcool comportant de 1 à 8 atomes de carbone.

9. Composition selon l'une quelconque des revendications précédentes, **caractérisée en ce qu'**elle comprend un ou plusieurs émulsionnants.

10. Composition selon la revendication précédente, **caractérisée en ce que** l'émulsionnant est choisi parmi les alkylpolyglucosides.

11. Composition selon l'une quelconque des revendications précédentes, **caractérisée en ce qu'**elle constitue un produit de soin de la peau ou un produit de maquillage ou un produit solaire.

12. Utilisation cosmétique de la composition selon l'une quelconque des revendications 1 à 10, pour le soin de la peau, et/ou pour le maquillage de la peau et/ou pour la protection solaire de la peau.

13. Utilisation cosmétique de la composition selon l'une quelconque des revendications 1 à 10, pour l'hydratation de la peau.

14. Utilisation cosmétique de la composition selon l'une quelconque des revendications 1 à 10, comme agent matifiant de la peau.

15. Utilisation cosmétique de la composition selon l'une quelconque des revendications 1 à 10, pour le traitement des peaux grasses.

16. Procédé de traitement cosmétique de la peau, destiné à lui apporter un aspect mat, à estomper les imperfections du relief de la peau, en particulier à camoufler les microreliefs, les rides et les ridules, les pores, **caractérisée en ce qu'**on applique sur la peau, une composition telle que définie selon l'une quelconque des revendications 1 à 10.

## Claims

1. Composition for topical application in the form of an oil-in-water emulsion comprising an oily phase dispersed in an aqueous phase, **characterized in that** it contains (1) at least 0.2% by weight, relative to the total weight of the composition, of one or more hydrophilic fumed silicas, and (2) at least one 2-acrylamido-2-methylpropanesulfonic acid homopolymer, and **in that** the aqueous phase represents at least 70% by weight relative to the total weight of the composition.

2. composition according to Claim 1, **characterized in that** it has a viscosity, measured at 25°C, of greater than 1 Pa.s, the viscosity being measured at 25°C with a Rheomat 180 measuring machine at 200 rpm.

3. Composition according to either of the preceding claims, **characterized in that** the amount of fumed silica(s) ranges from 0.2% to 4% by weight relative to the total weight of the composition.

4. Composition according to any one of the preceding claims, **characterized in that** the 2-acrylamido-2-methylpropanesulfonic acid homopolymer is a crosslinked and neutralized 2-acrylamido-2-methylpropanesulfonic acid homopolymer.

5. Composition according to any one of the preceding claims, **characterized in that** the 2-acrylamido-2-methylpropanesulfonic acid homopolymer is present in an amount ranging from 0.5% to 3% by weight relative to the total weight of the composition.

6. Composition according to any one of the preceding claims, **characterized in that** the amount of oily phase ranges from 1% to 30% by weight relative to the total weight of the composition.

7. Composition according to any one of the preceding claims, **characterized in that** the oily phase contains at least one volatile silicone oil.

8. Composition according to any one of the preceding claims, **characterized in that** the aqueous phase contains at least one alcohol containing from 1 to 8 carbon atoms.

9. Composition according to any one of the preceding claims, **characterized in that** it comprises one or more emulsifiers.

10. Composition according to the preceding claim, **characterized in that** the emulsifier is chosen from alkylpolyglucosides.

11. Composition according to any one of the preceding claims, **characterized in that** it is a skincare product, a makeup product or an antisun product.

12. Cosmetic use of the composition according to any one of Claims 1 to 10, for caring for the skin, and/or for making up the skin and/or for protecting the skin against the sun.

13. Cosmetic use of the composition according to any one of Claims 1 to 10, for moisturizing the skin.

14. Cosmetic use of the composition according to any one of Claims 1 to 10, as a skin matting agent.

15. Cosmetic use of the composition according to any one of Claims 1 to 10, for treating greasy skin.

16. Cosmetic skin treatment process, for giving the skin a matt appearance and for attenuating imperfections of the skin relief, in particular for covering the microrelief, wrinkles, fine lines and pores, **characterized in that** a composition as defined according to any one of Claims 1 to 10 is applied to the skin.

## Patentansprüche

1. Zusammensetzung zur topischen Anwendung in Form einer Ölin-Wasser-Emulsion, die eine in einer wässrigen Phase dispergierte Ölphase aufweist, **dadurch gekennzeichnet, dass** sie (1) mindestens 0,2 Gew.-% einer oder mehrerer hydrophiler pyrogener Kieselsäuren, bezogen auf das Gesamtgewicht der Zusammensetzung, und (2) mindestens ein 2-Acrylamido-2-methyl-propansulfonsäure- Homopolymer enthält, und **dadurch**, dass die wässrige Phase mindestens 70 Gew.-%, bezogen auf das Gesamtgewicht der Zusammensetzung, ausmacht.

2. Zusammensetzung nach Anspruch 1, **dadurch gekennzeichnet, dass** sie eine bei 25 °C bestimmte Viskosität über 1 Pa·s aufweist, wobei die Viskosität bei 25 °C mit einer Messvorrichtung Rheomat 180 bei 200 Umdrehungen pro Minute gemessen wird.

3. Zusammensetzung nach einem der vorhergehenden Ansprüche, **dadurch gekennzeichnet, dass** der Mengenanteil der pyrogenen Kieselsäure(n) im Bereich von 0,2 bis 4 Gew.-%, bezogen auf das Gesamtgewicht der Zusammensetzung, liegt.

4. Zusammensetzung nach einem der vorhergehenden Ansprüche, **dadurch gekennzeichnet, dass** das 2-Acrylamido-2-methyl-propansulfonsäure-Homopolymer ein vernetztes und neutralisiertes Homopolymer von 2-Acrylamido-2-methylpropansulfonsäure ist,

5. Zusammensetzung nach einem der vorhergehenden Ansprüche, **dadurch gekennzeichnet, dass** das 2-Acrylamido-2-methylpropansulfonsäure-Homopolymer in einer Menge von 0,5 bis 3 Gew.-%, bezogen auf das Gesamtgewicht der Zusammensetzung, enthalten ist.

6. Zusammensetzung nach einem der vorhergehenden Ansprüche, **dadurch gekennzeichnet, dass** der Mengenanteil der Ölphase im Bereich von 1 bis 30 Gew.-%, bezogen auf das Gesamtgewicht der Zusammensetzung, liegt.

7. Zusammensetzung nach einem der vorhergehenden Ansprüche, **dadurch gekennzeichnet, dass** die Ölphase mindestens ein flüchtiges Siliconöl enthält.

8. Zusammensetzung nach einem der vorhergehenden Ansprüche, **dadurch gekennzeichnet, dass** die wässrige Phase mindestens einen Alkohol mit 1 bis 8 Kohlenstoffatomen enthält.

9. Zusammensetzung nach einem der vorhergehenden Ansprüche, **dadurch gekennzeichnet, dass** sie einen oder mehrere Emulgatoren enthält.

10. Zusammensetzung nach dem vorhergehenden Anspruch, **dadurch gekennzeichnet, dass** der Emulgator unter den Alkylpolyglucosiden ausgewählt ist.

11. Zusammensetzung nach einem der vorhergehenden Ansprüche, **dadurch gekennzeichnet, dass** es sich um ein Produkt zur Pflege der Haut oder ein Produkt zum Schminken oder ein Sonnenschutzprodukt handelt.

12. Kosmetische Verwendung der Zusammensetzung nach einem der Ansprüche 1 bis 10 für die Pflege der Haut und/oder zum Schminken der Haut und/oder zum Sonnenschutz der Haut.

13. Kosmetische Verwendung der Zusammensetzung nach einem der Ansprüche 1 bis 10 für die Hydratisierung der Haut.

14. Kosmetische Verwendung der Zusammensetzung nach einem der Ansprüche 1 bis 10 als Mattierungsmittel für die Haut.

15. Kosmetische Verwendung der Zusammensetzung nach einem der Ansprüche 1 bis 10 zur Behandlung von fettiger Haut.

16. Verfahren zur kosmetische Behandlung der Haut, das dazu dienen soll, die Haut zu mattieren, Unzulänglichkeiten des Hautreliefs zu kaschieren und insbesondere Mikroreliefe, Falten und Fältchen und Poren zu verbergen, **dadurch gekennzeichnet, dass** auf die Haut eine Zusammensetzung, wie sie in einem der Ansprüche 1 bis 10 definiert ist, aufgetragen wird.
